# EUROPÄISCHE PATENTSCHRIFT

(11) **EP 1 757 562 B1**
(45) Veröffentlichungstag und Bekanntmachung des Hinweises auf die Patenterteilung: **23.07.2008**
(21) Anmeldenummer: 06115039.7
(22) Anmeldetag: 06.06.2006
(51) Int. Cl.: C02F 9/00, C02F 11/02

(54) **Anordnung und Verfahren zur Aufbereitung von Biogas-Gärresten, Güllen und Klärschlämmen**
Device and process for treating residues of biogas production, manure and sludge
Dispositif et procédé de traitement de lisier, de boues et de résidus de production de biogas

(30) Priorität: 07.06.2005 DE 102005026372; 07.01.2006 DE 102006000957
(43) Veröffentlichungstag der Anmeldung: 28.02.2007
(73) Patentinhaber: Richter, Daniela, 44263 Dortmund (DE); Hitze, Winfried, 48163 Münster (DE)
(72) Erfinder: Richter, Daniela, 44263 Dortmund (DE); Hitze, Winfried, 48163 Münster (DE)
(74) Vertreter: Siekmann, Gunnar

(56) Entgegenhaltungen:
- DE-A1- 3 943 416
- DE-A1- 4 131 604
- J. LEE ET AL.: "Tertiary treatment of biologically treated piggery wastewater using vibratory shear-enhanced RO membrane" WATER SCIENCE AND TECHNOLOGY, Bd. 49, Nr. 5-6, 2004, Seiten 435-442, XP008073797

## Beschreibung

Die vorliegende Patentanmeldung betrifft eine Anordnung und ein Verfahren zur Aufbereitung von Biogas-Gärresten, Güllen und Klärschlämmen. Die Anmeldung nimmt die Priorität der DE 10 2005 026 372 sowie der DE 10 2006 000 957 in Anspruch, deren Offenbarungsgehalt vollumfänglich in die vorliegende Patentanmeldung einbezogen wird. Der Inhalt der beiden Anmeldungen findet sich in den als Bestandteil der vorliegenden Anmeldung eingereichten Anhängen. Dabei wurden die Abbildungen der beiden älteren Anmeldungen zur Vermeidung von Verwechselungen umnummeriert .

Betreiber von Biogasanlagen sehen sich häufig dem Problem gegenüber, dass sie große Mengen an zu entsorgenden Gärresten erzeugen, für die in der Regel ein Flächennachweis erbracht werden muss. Ähnliches gilt für die Gülleproduktion in landwirtschaftlichen Betrieben sowie die Produktion von Klärschlamm.

Da die genannten Materialien hohe Wassergehalte aufweisen, sind die zu entsorgenden Volumina sehr groß. Im Laufe der vergangenen Jahre wurden daher zahlreiche Verfahren entwickelt, um das Volumen des zu entsorgenden Materials durch Wasserentzug zu verringern und das gewonnene Wasser gleichzeitig zu reinigen, so dass es ohne weiteres eingeleitet werden kann.

Die Aufbereitung von Güllen, Gärresten aus der Biogasproduktion und Klärschlämmen mittels mechanischer Vorreinigung und nachgeschalteter Membranfiltration - letztere gestuft von der Ultrafiltration bis zur Umkehrosmose - ist Gegenstand vieler Veröffentlichungen sowie von technischen Schutzrechten.

So beschreibt beispielsweise die EP 1211308 eine Anordnung zur Aufbereitung von Gärresten mit mehrstufiger Filtration. Dabei erfolgt die Aufbereitung in einer Schneckenpresse, in welcher das zu filtrierende Material einem Druck ausgesetzt wird. Die EP 1072574 beschreibt eine Vergärungsanlage mit einem Fermenter und einer Entwässerungsvorrichtung. Als letztere kommt dabei z.B. eine Schneckenpresse zum Einsatz. Die DE 3941016 betrifft Zyklonfilter, die in der hydraulischen Ausrüstung von Stationäranlagen und in Kraftfahrzeug- und Traktorhydrosystemen zur Reinigung von Druck-, Öl- und Kühlflüssigkeiten von mechanischen Fremdstoffen verwendet werden. Dasselbe gilt für die DE 29901346.

Das bereits erteilte Patent DE 10 2004 030482 der Erfinder der vorliegenden Anmeldung betrifft ein Verfahren zur Aufbereitung von Abwässern aus der Bearbeitung und Aufbereitung von organischen Abfällen, insbesondere von Gülle, mit einem Biogasfermenter, mit daran anschliessender Ultrafiltration und Umkehrosmose. Dabei wird bei der Ultrafiltration abgetrennter Schwebtrub in den Biogasfermenter rückgeführt.

Die Offenlegungsschrift DE-A-4 131 604 beschreibt ein Verfahren zur Aufbereitung von Klärschlämmen mittels horizontaler Querstrommikrofilter, bei denen eine semipermeable Membran einen um eine Achse rotierenden Rotationskörper in geringem radialem Abstand umschließt.

Auch die Aufbereitung von Klärschlämmen aus der Abwasseraufbereitung in Kläranlagen mittels Flockung, mechanischer Trennung, Wassernachfiltration oder Wasser-rückführung ist in der Praxis bereits eingesetzt.

Nun weisen gerade Güllen, Biogas-Gärreste und Klärschlämme hohe Anteile stark haftender organischer Schwebstoffe auf; erstere enthalten überdies reichlich Kalzium und Sulfat, was zu

Schwebkalk und Gips führt. Gerade diese Gehalte führen in allen Membranfiltersystemen zu einer starken Verschmutzung und Verstopfung der Filterporen (Scaling) sowie zur Ausbildung von Mikroorganismenbewuchs (Fouling).

Deshalb wird heute in der mechanischen Reinigungsstufe durch entsprechende Geräte im Kaskadeneinsatz ein wesentlich verbesserter Austrag von organischen Schwebstoffen angestrebt, so z. B. mit Siebschneckenseparatoren in Grob- und Feinsiebausstattung oder mit Dekantern. Dabei kommen häufig Flockmittel zum Einsatz, wie z.B. AlCl₃, Eisen-III-Chlorid oder Acrylate. Diese sind jedoch für bestimmte Einsatzzwecke nicht erwünscht, da deren Verwendung einerseits mit Kosten verbunden ist, und andererseits insbesondere dann, wenn eine der durch die Trennung erhaltenen Fraktionen als Düngemittel verwendet werden soll.

Doch trotz dieser wesentlich verbesserten mechanischen Aufbereitung kann das so aufbereitete Gülle- oder Gärrest- oder Klärschlammprodukt nicht direkt auf Röhrenmembranen im Nano- oder Umkehrosmosebereich gegeben werden, um ein einleitfähiges Restwasser zu erhalten, sondern es wird zunächst eine Ultrafiltration mit entsprechender Überströmung durchgeführt. Filtersysteme, deren Membranen allein durch kräftiges Überströmen vor vorzeitiger Verblockung geschützt werden können, sind für die Klärschlamm-, Gülle- und Gärrestaufbereitung weitgehend ungeeignet.

Jede Verfahrenstechnik - und das gerade in der Klärschlamm-, Gülle - und Gärrestaufbereitung - muss jedoch ebenso energiewirtschaftlich geprüft werden. Hier zeigt sich, dass die klassischen mechanischen Trennsysteme und die mit hoher Überströmung arbeitenden Filtersysteme alle sehr energieintensiv sind, so dass allein für die mechanische Bearbeitung von einem Kubikmeter obiger Produkte über 2 kW Energie eingesetzt werden müssen. In etwa den gleichen Energiebetrag benötigt die Filtertechnik. Aufgrund der hohen Komplexität sind die genannten Systeme zudem sehr fehleranfällig.

Hinzu kommt, dass bei den genannten Verfahren die Phosphataustragung nicht immer kontrollierbar bzw. nicht immer in die am besten geeignete Fraktion erfolgt. Dies ist deswegen von Bedeutung, weil die Rohstoffpreise für Phosphat aufgrund erschöpfter Lagerstätten ansteigen, und es daher wirtschaftlich sinnvoll sein kann, durch gezielten Austrag des Phosphatgehalts eines Gärrückstandes, eines Klärschlamms oder einer Gülle einen wirtschaftlich attraktiven Phosphatdünger zu gewinnen.

Aufgabe der vorliegenden Erfindung ist es daher eine Anordnung und ein Verfahren zur Aufbereitung von Biogas-Gärresten, Güllen und Klärschlämmen bereitzustellen, die mit geringem Energieaufwand eine effiziente Aufbereitung der betreffenden Materialien ermöglicht, und gleichzeitig eine hohe Betriebssicherheit und Zuverlässigkeit aufweist.

Diese Aufgabe wird mit den Merkmalen der vorliegenden unabhängigen Ansprüche gelöst.

Demnach ist ein Verfahren zur Aufbereitung von Klärschlämmen, Güllen und Gärresten aus der Biogasproduktion, das die folgenden Schritte aufweist:
a. Einbringen des Klärschlamms, der Gülle oder des Gärrests in einen Feinsiebunterdruckseparator (FUS), aufweisend eine Senkrecht-Hebewendel, einen die Wendel umschließenden Zylinder, der zumindest abschnittsweise ein Sieb mit einer Maschengröße zwischen 30 und 300 µm aufweist, sowie ein den Zylinder umgebendes Unterdruckgehäuse,
b. Herstellen und Auffangen eines Filtrats sowie eines separierten Feststoffs,
c. Einbringen des Filtrats in einen Vibrationsscherkraftfilter und
d. Herstellen und Auffangen eines primären Permeats sowie eines primären Retentats.

In dem vorliegenden Verfahren wird erstmals ein Feinsiebunterdruckseparator zur Aufbereitung von Biogas-Gärresten, Güllen und Klärschlämmen verwendet. Solche Feinsiebunterdruckseparatoren sind erst seit kurzem auf dem Markt und daher in gattungsgemäßen Verfahren noch nicht zum Einsatz gekommen. Bei einem solchen FUS-Gerät handelt es sich um eine Wendelfilterpresse, die unter Unterdruck betrieben wird. Der Begriff "Presse" ist daher im eigentlichen Sinne unzutreffend, da das Filtrat nicht durch Pressen gewonnen wird. Die Filtration erfolgt seitens der Hebewendel drucklos, da die die Filtration treibende Kraft einerseits die Schwerkraft und andererseits das von außen anliegende, in dem den Zylinder umgebenden Unterdruckgehäuse etablierte Vakuum ist. Eine Wendelfilterpresse ist daher nicht mit den herkömmlichen Schneckenpressen vergleichbar.

Aus diesen Gründen wird in der vorliegenden Anmeldung anstelle des Begriffs Wendelfilterpresse der Begriff Feinsiebunterdruckseparator verwendet.

Bevorzugt weist das Sieb des die Wendel umschließenden Zylinders eine Maschengröße zwischen 30 und 150 µm auf. Bevorzugt verwendete Maschengrößen liegen bei 50, 75, 100 und 130 µm.

Weiterhin ist der gesamte Zylinder des Feinsiebunterdruckseparators bevorzugt in Form eines Zylindersiebs ausgestaltet. Dabei wird durch das Sieb ein permanenter Sog von innen nach außen angelegt. Hierfür kann z.B. eine Seitenkanalpumpe verwendet werden, jedoch auch eine Kombination aus einer Förderpumpe (z.B. einer Kreiselpumpe und einer Vakuumpumpe, z.B. einer Membranpumpe). Es wird dabei bevorzugt ein konstanter Unterdruck zwischen -0,1 bar und -0,4 bar an der Siebaußenseite angelegt.

Die vorliegende Erfindung schlägt erstmals die Verwendung solcher Feinsieb-Separatoren für die Aufarbeitung von Biogas-Gärresten, Güllen und Klärschlämmen vor. Gerade der Einsatz der Hebewendel als Presswendel würde das mit Feinsieb ausgestattete Gerät für den obigen Einsatz untauglich machen, da es sehr schnell zu einer Verstopfung der Siebmaschen käme und die mechanische Belastung und der damit einhergehende Verschleiß der Siebe überaus groß wäre. Die Erfinder haben nun überraschender Weise gezeigt, dass unter den vorgeschlagenen Bedingungen im Unterdruck, insbesondere bei den oben genannten Druckwerten, eine effektive Erstfiltration von Biogas-Gärresten, Güllen und Klärschlämmen mit Feinsieb-Separatoren möglich ist.

Die in dem Feinsiebunterdruckseparator eingebaute Wendel ist deshalb bevorzugt keine Presswendel, da diese die Siebporung verstopfen oder zuviel Faserstoffe durch das Sieb drücken würde, sondern eine Senkrecht-Hebewendel, die den Klärschlamm, die Gülle oder den Gärrest gleichmässig von unten nach oben an der Siebzylinder-Innenseite entlang führt. Dabei wird der wässrige Extrakt mit den Partikeln, die das Sieb passieren können, systematisch abgesaugt und gleichzeitig der verbliebene Rückstand innerhalb der aufsteigenden Wendelnut systematisch schüttfähig gemacht und über den Wendelkopf ausgeworfen. Die Hebewendel kann dabei durch unterschiedliche Nuttiefen und durch eine unterschiedliche Anzahl der Wendelzüge direkt an die Beschaffenheit des zu filtrierenden Materials angepasst werden. Zum Einsatz kommen dabei bevorzugt zwischen 2 und 10 Wendelzüge, besonders bevorzugt zwischen 3 und 5 Wendelzüge.

Die Hebewendel weist bevorzugt eine Höhe von 20 - 90 cm auf. Weiterhin kann vorgesehen sein, dass das Sieb des Feinsiebunterdruckseparators mechanisch abgestützt ist und/oder eine Oberflächenbeschichtung, bevorzugt aus PTFE, (Polytetrafluorethylen, Handelsname Teflon) aufweist.

Weist das Sieb eine sehr geringe Maschengröße auf (z.B. geringer als 80 µm), droht die Gefahr der Belagbildung auf der Siebinnenwand; dies insbesondere dann, wenn das Material bereits vorfiltriert war. In diesem Falle fehlen nämlich dem Material gröbere Bestandteile, wie z.B. Fasern, die eine Reinigungswirkung auf das Sieb ausüben und so Verstopfungen und Beläge verhindern. Überdies läßt sich ein z.B. mit einem 100 µm-Sieb bereits filtriertes "glattes" Gülle-, Klärschlamm- oder Gärrestfiltrat aufgrund eines wässrig-öligen Aggregatzustandes von der Hebewendel wesentlich schlechter nach oben transportieren.

Für solche Fälle ist daher bevorzugt vorgesehen, dass die Gülle-, Klärschlamm- oder Gärrestfiltratzuführung zu dem Feinsiebunterdruckseparator im geschlossenen System mittels abgestimmter Pumpleistung zur Unterstützung der Hebewendel erfolgt. Zusätzlich kann die Hebewendel mit nachstell- und auswechselbaren Außenkanten-Abstreiflippen versehen sein, die eine Belagbildung an der Filterinnenfläche verhindern. Außerdem kann die Saugleistung dem erhöhten Siebdurchgangswiderstand angepasst und/oder die Porenausgänge an der Siebaußenwandung können mittels pulsierendem Ultraschall gereinigt werden.

Ein solcher Feinsiebunterdruckseparator mit einem 100 µm-Zylindersieb kann bis zu 600 Liter Gülle, Gärrest oder Klärschlamm pro Stunde verarbeiten, wobei der hier anfallende Feststoffaustrag in Abhängigkeit von der verwendeten Hebewendel zwischen 42 und 52 % des gesamten Trockenmasseangebots dieser Charge enthält. Das ausgetragene schüttfähige Feststoffmaterial hat einen Anteil an organischer Trockensubstanz (OTS) von 22 - 30 %.

Bei Nachschaltung eines weiteren Feinsiebunterdruckseparators, der beispielsweise mit einem 50 µm-Sieb bestückt ist, lassen sich weitere 25 - 35 % der im Filtrat verbliebenen Trockenmasse in die Feststoffphase überführen.

Durch Parallelschaltung mehrerer Feinsiebunterdruckseparatoren läßt sich der stündliche Durchsatz an Gülle, Gärrest oder Klärschlamm weiter erhöhen.

Bei dem erfindungsgemäß verwendeten, dem FUS nachgeschalteten Vibrationsscherkraftfilter handelt es sich bevorzugt um ein sogenanntes VSEP-System (Vibratory Shear Enhanced Processing). Solche Geräte werden z.B. von der Firma New Logic hergestellt und vertrieben und beruhen auf dem Prinzip, eine Filtermatrix unter Vibrationen zu setzen und so eine Verstopfung der Filterporen zu vermeiden.

Hervorstechendes Merkmal solcher VSEP-Systeme, die zunächst zur Abtrennung von Mineralölresten aus Seewasser entwickelt wurden, ist, dass das gesamte mit Filtriergut befüllte System mit einer bestimmten Frequenz und Amplitude schwingt und damit eine Partikelverblockung der Membranen effektiv verhindert. Während bei herkömmlichen Filtersystemen eine Überspülung der Filtermatrix zur Verhinderung von Verstopfungen derselben erforderlich ist, kann bei dem erfindungsgemäß eingesetzten VSEP-System auf eine solche Überspülung verzichtet werden. Die gesamte Pumpenleistung liegt in dieser Technik entsprechend niedrig. Damit beträgt der gesamte Energieaufwand pro Volumeneinheit Permeat nur rund 20 % des Energieaufwandes, den z.B. eine Anlage mit Röhrenmembranen gleicher Porung für dasselbe Volumen benötigt.

In dem vorliegenden Verfahren wird ein Vibrationsscherkraftfilter erstmals zur Aufbereitung von Biogas-Gärresten, Güllen und Klärschlämmen verwendet.

Die Kombination aus FUS und VSEP ist ebenfalls bislang noch nicht vorgeschlagen worden. Dabei haben die Erfinder erstmals gezeigt, dass sich beide Systeme ideal ergänzen. Da das VSEP-System in Grenzbereich zwischen Nanofiltration und Umkehrosmose eingesetzt wird, ist es auf große Mengen relativ gut vorgereinigter Substrate, so wie sie die erfindungsgemäß bvetriebenen FUS-Geräte zur Verfügung stellen.

Das VSEP-Prinzip kann für verschiedene Filtrationstypen verwendet werden, die sich je nach Ausschlußgröße voneinander unterscheiden.

| **Typ** | **Ausschlußgröße** | **Molekulargewicht** |
|---|---|---|
| Umkehrosmose | ≤ 0.001 | ≤ 100 Dalton |
| Nanofiltration | 0.001-0.01 µm | 100-1000 Dalton |
| Ultrafiltration | 0.01-0.1 µm | 1-500 kD |
| Mikrofiltration | ≥ 0.1 µm | ≥ 500 kD |

Der in dem erfindungsgemäßen Verfahren verwendete Vibrationsscherkraftfilter weist deshalb bevorzugt eine Ausschlußgröße zwischen 30 und 300 Dalton auf. Der Verfahrensschritt bewegt sich damit im Grenzbereich zwischen Umkehrosmose und Nanofiltration bzw. liegt direkt im Umkehrosmosebereich. Der Filter wird bevorzugt mit einer Vibrationsfrequenz zwischen 10 und 100 Hz sowie einer Amplitude zwischen 5 und 50 mm betrieben.

Die Auswahl der Membranart und der Filterflächengröße erfolgt in Anpassung des Verfahrens an den Volumenstrom und an die Art und Menge der zugeführten Stoffe in dieser Aufbereitungsstufe. Sowohl der Betrieb direkt als Umkehrosmose wie auch als Nanofiltration sind jeweils ein Novum in der Gülle-, Gärrest und Klärschlammaufbereitung.

In einer bevorzugten Ausgestaltung des erfindungsgemäßen Verfahrens ist vorgesehen, dass das Filtrat nach Passieren des Feinsiebunterdruckseparators und vor Einbringen in den Vibrationsscherkraftfilter in einen weiteren Feinsiebunterdruckseparator eingebracht wird, wobei letzterer zumindest abschnittsweise ein Sieb mit einer geringeren Maschengröße aufweist als der erstgenannte Feinsiebunterdruckseparator.

Auf diese Weise läßt sich z.B. eine Kaskade aus einem 100 µm-Zylindersieb und einem 75 µm- oder 50 µm-Zylindersieb herstellen. Letzteres ermöglicht einen weiteren Trockenmasse-Austrag von ca. 25 % bis zu ca. 35 % aus dem Erstfiltrat (auch diese %-Werte sind auf die Ausgangstrockenmasse = 100 % bezogen).

Es lassen sich mit diesen Verfahrensschritten bereits zwischen 70 % und 80 % der Gesamttrockenmasse aus Güllen, Klärschlämmen und Gärresten isolieren. Auf diese Weise wird erstmals die direkte Weiterverarbeitung der Filtrate (ohne den bisher erforderlichen Zwischenschritt der Ultrafiltration) in der Nano- oder Umkehrosmosetechnik ermöglicht.

Hinzu kommt, dass sich die FUS-Geräte leicht in Doppelreihe oder auch im Kreis anordnen lassen. Die Geräte sind typische Langsamläufer, robust im Aufbau, einfach zu bedienen sowie selbstreinigend.

Verfahrenstechnisch aber ebenso wichtig ist, dass pro Kubikmeter Gülle-, Gärrest- oder Klärschlamm-Ausgangsmaterial nur 0,5 kW bis max. 0,6 kW benötigt werden, was die FUS-Geräte als sehr energiesparend auszeichnet.

Ein weiterer Vorteil dieser mechanischen FUS-Technik bei Güllen und vor allem bei Gärresten ist, dass ein mit einem 100 µm-Zylindersieb bestücktes FUS-Gerät weniger als 20 % des Gesamtphosphats mit dem Erstaustrag (Feststoff) auswirft, während z.B. bei einem Dekanter über 50 % und bei Siebpressschnecken rund 30 % des Gesamtphosphats mit dem Erstaustrag (Feststoff) ausgeworfen werden.

Bei einem kaskadierten FUS-System, in welchem in der zweiten Stufe ein 75 µm bzw. 50 µm-Zylindersieb verwendet wird, kann bei wesentlich geringerer Organofracht in dem zu filtrierenden Material mehr Phosphat ausgetragen werden, und zwar 50 % bis 60 % des Gesamtphosphats. Diese Charge kann getrocknet und direkt zu Organo-Phosphat-Dünger aufgearbeitet werden, oder sie wird einem phosphatarmen Flüssigkonzentrat aus der Nano/Umkehrosmosetechnik zugemischt und anschließend zu Organo-NPK-Dünger aufgearbeitet.

Mithilfe des zweistufigen FUS-Verfahrens lassen sich also gezielt Fraktionen mit definiertem Phosphatgehalt herstellen.

Abschließend sei im Hinblick auf den Erstaustrag aus dem mit einem 100 µm-Sieb bestückten FUS-Gerät auf folgende drei Aspekte verwiesen:

Bei Verarbeitung von Gülle mit dem erfindungsgemäßen Verfahren fällt direkt auf dem Hof ein Güllefeststoff an, der sich für den Einsatz in Biogasanlagen eignet, und bei vergleichbarer Masse einen Biogasertrag von 50 % im Vergleich zur Maissilage bringt. Neben der Einsparung von inzwischen teurer Maissilage ermöglicht ein solcher Güllefeststoff eine wesentlich verbesserte Gärraumauslastung. Gleichzeitig senkt der Einsatz von Betriebswasser statt Gülle zusätzlich den schädlichen Ammoniak-/Ammoniumeintrag in den Fermenter.

Für den mechanisch ausgetragenen Gärrest-Feststoff zeigt die Praxis verschiedene Anwendungen auf. Neben der Herstellung von Pellets und Briketts als Brennstoff kann das Material getrocknet und als Einstreu eingesetzt oder zu Humus aufgearbeitet werden. Überdies kann es hygienisiert, vorgetrocknet und zu stapelbaren großen Blöcken gepresst raumsparend verschifft werden, und, z.B. in Übersee, als Bodenverbesserer verwendet werden.

Das gilt ebenso für erfindungsgemäß hergestellten hygienisierten, schadstofffreien Feststoff aus Klärschlamm, der aber allein oder zusammen mit Strohfeinschnitt zu Briketts verarbeitet auch Heizstoff und damit Wärmequelle für die Organo-Trockendüngerherstellung sein kann. Beide Produkte beinhalten viel Phosphat, so dass die hieraus gewonnene Asche, auf Schadstoffe geprüft, einen verwertbaren Phosphatdünger ergibt.

Untersuchungen der Anmelder haben ergeben, dass allein durch die FUS-Technik im 2-stufigen Verfahren selbst Rindergüllen mit bis zu 12 % Trockenmasse (TM) auf bevorzugt unter 3,5 % TM herabgesetzt und Güllen, Gärreste sowie Klärschlämme mit wesentlich weniger TM bereits auf unter 2,5 % TM herabgesetzt werden können.

Im Prinzip ist das mit Hilfe des VSEP-Schritts erhaltene primäre Permeat bereits soweit geklärt, dass es in einen Vorfluter eingeleitet werden kann, da das VSEP-System wie erwähnt als Umkehrosmosesystem betrieben werden kann. Die Annmelder haben in Untersuchungen festgestellt, dass sich der Ammonium-Gehalt bereits mit diesen Verfahrensschritten auf weniger als 15 mg/l Permeat senken lassen kann. Gleichwohl kann ggf. aufgrund nationaler Einleitungsbestimmungen ein weiterer Reinigungsschritt erforderlich sein, insbesondere um den Ammoniumgehalt des Permeats unter die Nachweisgrenze zu drücken.

Aus diesem Grunde ist in einer besonders bevorzugten Ausgestaltung des erfindungsgemäßen Verfahrens vorgesehen, dass das primäre Permeat aus dem Vibrationsscherkraftfilter einer Umkehrosmose unterzogen wird, und dass das auf diese Weise hergestellte sekundäre Permeat sowie des sekundäre Retentat aufgefangen werden.

Die nachgeschaltete Umkehrosmose kann einstufig, aber auch zwei- oder dreistufig sein. Dabei kann das mit Inhaltsstoffen stärker angereicherte Retentat aus der ersten Umkehrosmosestufe dem VSEP-Grundretentat (primäres Retentat) zugeführt werden. Das zweite und das eventuell dritte Retentat aus der zweiten und dritten Umkehrosmosestufe werden in das Ausgangsmaterial vor der mechanischen Aufbereitung oder wahlweise in die erste Umkehrosmosestufe rückgeführt.

Besonders bevorzugt ist vorgesehen, dass das Filtrat und/oder das bzw. die Permeate angesäuert werden. Durch diesen Schritt wird im Permeat enthaltenes Ammonium zu Ammoniumsulfat umgesetzt, das im Gegensatz zu ersterem mit den genannten Reinigungsverfahren (VSEP, Umkehrosmose) gut aus der wässrigen Phase entfernt werden kann. Das erhaltene Permeat wird anschließend bevorzugt neutralisiert, insbesondere durch Passage durch einen Kalksteinfilter.

In einer besonders bevorzugten Ausgestaltung des erfindungsgemäßen Verfahren ist, wie z.T. schon erwähnt, vorgesehen, dass der separierte Feststoff als Bodenverbesserer, als Düngemittel, als Brennstoff, als Gärsubstrat, als Zellulose-Füllstoff, als ein Bestandteil derselben oder zur Herstellung derselben verwendet wird.

Das primäre und/oder sekundäre Retentat werden bevorzugt als Düngemittel weiterverwendet bzw. zu einem Handels-Düngemittel weiterverarbeitet.

Ebenso bevorzugt werden der separierte Feststoff und das primäre und/oder sekundäre Retentat vereinigt und als Bodenverbesserer, als Düngemittel, als Brennstoff, als Gärsubstrat, als mineralischer Dünger, als ein Bestandteil derselben oder zur Herstellung derselben verwendet.

Das primäre und/oder sekundäre Permeat werden bevorzugt als Betriebswasser in einer Biogasanlage, als Reinigungs- oder Kühlwasser oder als landwirtschaftliches Produktionswasser verwendet und/oder in einen Vorfluter, einen Bach, einen Wasserlauf oder eine Kanalisation eingeleitet.

Nochmals zusammengefasst führt das gesamte Verfahren zu folgenden Austrägen und Produkten:
Im ersten mechanischen Austrag mittels Feinsiebunterdruckseparator mit 100 µm Zylindersieb erhält man:
   a) bei Gülleaufbereitung einen Feststoff, der so belassen direkt im Biogas-Fermenter verarbeitet werden kann,
   b) bei Gärrestaufbereitung einen phosphatarmen Feststoff, der entsprechend aufgearbeitet (wie beschrieben) ein guter Bodenverbesserer ist,
   c) bei Klärschlamm für die Landwirtschaft einen phosphatreichen Feststoff, der ebenso aufgearbeitet ein Bodenverbesserer ist, aber auch zusammen mit Strohpellets als Heizmaterial eingesetzt werden kann, wobei die anfallende Asche phosphatreich ist und als Dünger verwendet werden kann.

Im zweiten mechanischen Austrag mittels Feinsiebunterdruckseparator mit 75 µm- oder 50 µm- Zylindersieb erhält man eine an Phosphat angereicherte Charge, die komplett dem VSEP-Konzentrat zugemischt wird, alternativ wird diese Charge zu Organo-Phosphat-Dünger aufgearbeitet, z.B. mit der oben erwähnten phosphathaltigen Asche.

Im VSEP-System wird aus Güllen oder aus Gärresten ein Konzentrat hergestellt, das Rohstoff für die Organo-NK- (Organo-Stickstoff-Kali-) oder Organo-NPK- (Organo-Stickstoff-Phosphor-Kali-) Düngerherstellung ist.

Ohne jede weitere Düngemittelaufbereitung liefert das angezeigte Verfahren einen streufähigen Organo-Phosphatdünger sowie ein Organo-NK-Düngerkonzentrat, die zusammen ein Organo-NPK-Düngerkonzentrat zur weiteren Aufarbeitung ergeben. Hierbei handelt es sich um Wirtschaftsdünger, die unter Nutzung der BHKW-Abwärme oder einer anderen Wärmequelle aus der Verbrennung von Pellets und Briketts aus Stroh nach Marktbedarf in Handelsdünger überführt werden können, um damit die Produkthygienisierung, die Produktstabilisierung, alle sonstigen rechtlichen Anforderungen und eine gute Düngerausbringung auf dem Acker zu gewährleisten.

Dazu bedarf es für flüssige Dünger zur Hygienisierung und weiterer Einengung auf konstante Dünger-Mindestgehalte der Nachbereitung im Verdampfer. Sie können aber im Verdampfer auch soweit eingeengt werden, dass sie vom Schnecken-, Schaufel- oder Bandtrockner direkt übernommen werden können. Sie werden hierin auf Wassergehalte von 20 % bis 13 %, bevorzugt um 15 %, getrocknet und direkt einheitlich granuliert. Hierfür muss Flüssigdünger grundsätzlich zunächst auf einen Wassergehalt unter 60 % eingeengt werden.

Alle bereits schüttfähigen Phosphatwirtschaftsdünger werden direkt in der Trocknungsanlage aufgetrocknet und vermahlen bzw. granuliert. Neben Granulatdünger ist auch der pelletierte Dünger in der Praxis sehr gefragt. Dazu wird der feuchte Phosphatdünger mit Phosphatasche aus der Klärschlamm-Strohverbrennung vermischt und mit feinem trockenen Strohhäcksel auf einen Wassergehalt von unter 60 % gebracht, die Masse wird abgebunden und pelletiert und die Phosphatpellets danach getrocknet.

Mit der Düngerherstellung aus Güllen und aus Gärresten sind zwei spezifische Probleme verbunden: Beim Erhitzen der Flüssig- und Feuchtware ist mit einem erheblichen Ammoniakaustrag dann zu rechnen, wenn die Produkte nicht genügend angesäuert wurden. Deshalb ist die gezielte Säuerung und Ammoniumsulfatbildung ein bevorzugtes Merkmal des gesamten Verfahrens. Außerdem wird zusammen mit dem Wasserdampf ein hohes Potential an Geruchsstoffen ausgetragen. Daher wird in einer bevorzugten Ausgestaltung die gesamte Nassluft über einen Luftwäscher geführt. Hieran schließt sich bevorzugt eine Luftkühlung mit Wasserkondensierung mittels Betriebswasser als Kühlmittel und die Führung der gekühlten Luft über einen Biofilter an.

Das Kondensat kann der Nutzung als Betriebswasser direkt zugeführt werden. Um es einleitfähig zu machen, wird das Kondensat der Umkehrosmose zugeführt.

Weiterhin ist erfindungsgemäß eine Vorrichtung zur Durchführung eines der obigen Verfahren vorgesehen, mit mindestens einem Feinsiebunterdruckseparator (FUS), aufweisend eine Senkrecht-Hebewendel sowie einen die Wendel umschließenden Zylinder, der zumindest abschnittsweise ein Sieb mit einer Maschengröße zwischen 30 und 300 µm aufweist, sowie ein den Zylinder umgebendes Unterdruckgehäuse,
zur Auftrennung von Klärschlamm, Gülle oder eines Gärrests in einen Feststoff und ein Filtrat, sowie einem Vibrationsscherkraftfilter zur Auftrennung des Filtrats in ein erstes Permeat und ein erstes Retentat.

Bevorzugt weist das Sieb eine Maschengröße zwischen 30 und 150 µm auf. Bevorzugt verwendete Maschengrößen liegen bei 50, 75, 100 und 130 µm.

Dabei ist besonders bevorzugt mindestens ein weiterer Feinsiebunterdruckseparator vorgesehen, der zumindest abschnittsweise ein Sieb mit einer geringeren Maschengröße aufweist als der erstgenannte Feinsiebunterdruckseparator.

Hinzu kommt bevorzugt eine Umkehrosmoseeinrichtung zur Auftrennung des ersten Permeats in ein zweites Permeat und ein zweites Retentat.

Etwaige bevorzugte Ausführungsformen der erfindungsgemäßen Vorrichtung sind bereits bei den betreffenden Verfahrensansprüchen diskutiert. Es wird daher auf die obige Diskussion verwiesen.

Die vorliegende Erfindung wird durch die im Folgenden gezeigten und diskutierten Figuren genauer erläutert. Dabei ist zu beachten, dass die Figuren nur beschreibenden Charakter haben und nicht dazu gedacht sind, die Erfindung in irgendeiner Form einzuschränken.

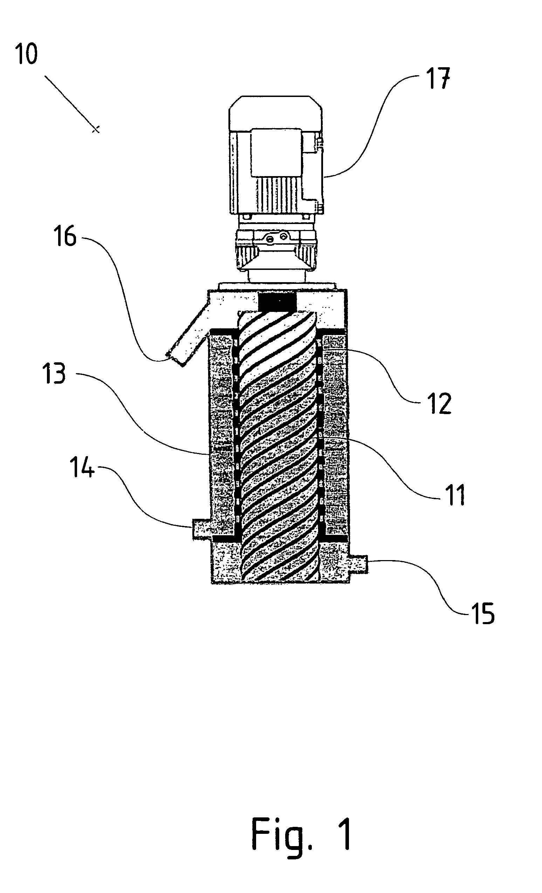

Fig. 1 zeigt einen Feinsiebunterdruckseparator 10 mit einer druckfreien Hebewendel 11, die in einem Siebzylinder 12 angeordnet ist. Die Siebe des Letzteren weisen eine Maschenweite von 100 µm auf. In alternativen Ausführungsformen sind Maschengröße zwischen 30 und 150 µm vorgesehen.

Der Siebzylinder 12 ist in einer Unterdruck-Ringkammer 13 angeordnet, an welcher über einen Vakuumstutzen 14 ein Vakuum angelegt wird, beispielsweise mittels einer nicht dargestellten Seitenkanalpumpe. Über diesen Kanal wird auch das durch den Siebzylinder hindurchgetretene Filtrat abgesaugt. Die

Filtration erfolgt also seitens der Hebewendel drucklos, wobei die treibende Kraft für die Filtration einerseits die Schwerkraft und andererseits das von außen anliegende Vakuum ist. Über einen Einlaufstutzen 15 wird der aufzubereitende Gärrest, Klärschlamm bzw. die Gülle eingebracht. Die durch die Hebewendel nach oben transportierten Feststoffe werden durch den Feststoffaustragstutzen 16 ausgetragen. Die Hebewendel wird durch einen Antrieb 17, der Dauerlaufeigenschaften aufweist (in Abb. 1 als Elektromotor dargestellt), angetrieben.

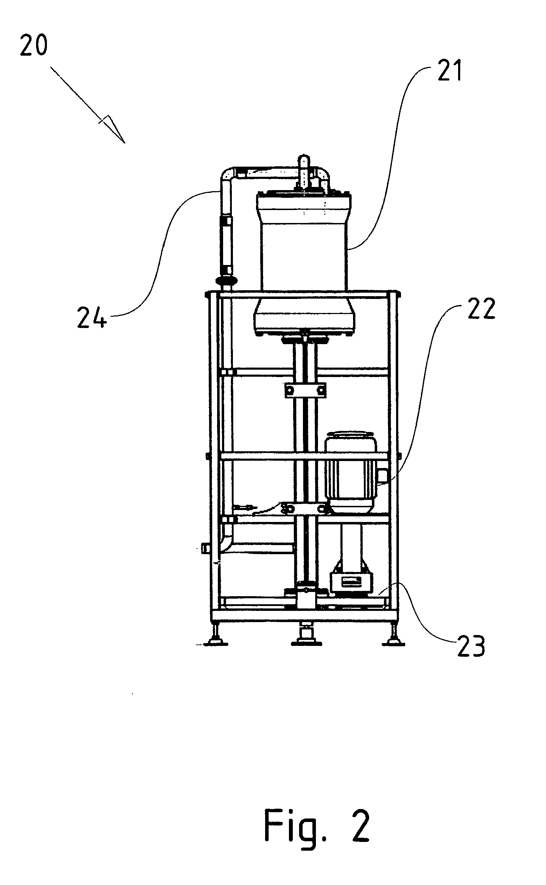

Fig. 2 zeigt ein Vibrationsscherkraftfiltersystem 10, wie es z.B. unter dem Begriff VSEP-System (Vibratory Shear Enhanced Processing) von der Firma New Logic hergestellt und vertrieben wird, mit einem Filterpack 21 und einem Antriebsmotor 22. Der Antriebsmotor 22 überträgt eine Drehbewegung auf ein exzentrisch angeordnetes Gewicht 23, das wiederum das ganze System in eine horizontal gerichtete Scherbewegung versetzt. Oberhalb des Filterpacks 21 ist ein Einlassrohr 24 für das zu filtrierende Material angeordnet. Die Auslassrohre für das Retentat und das Permeat sind in Fig. 2 nicht dargestellt.

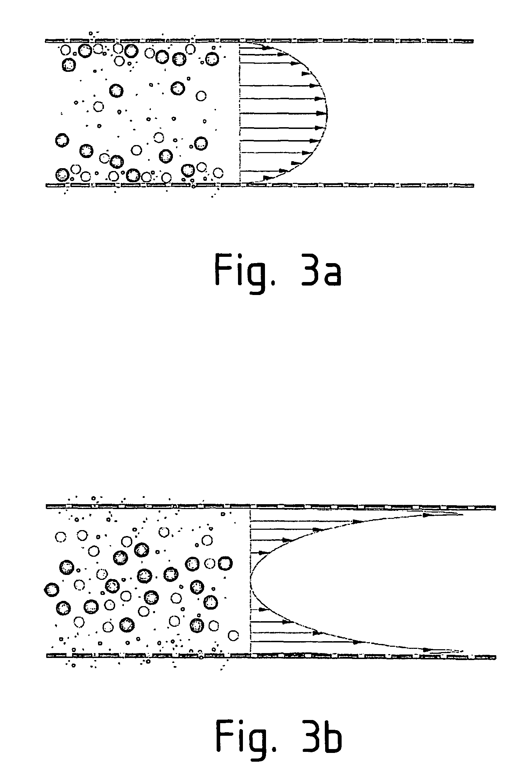

Fig. 3a zeigt das Funktionsprinzip eines herkömmlichen Crossflow-Membranfiltersystems, bei dem die Verblockung der Filtermembran mit Hilfe einer Überströmung verhindert werden soll. Aufgrund hydrodynamischer Gegebenheiten stellt sich in einem solchen System ein parabolisches Strömungsprofil ein, mit der Folge, dass im Bereich der Ränder, also dort, wo eine möglichst starke Strömung notwendig wäre, um eine Verblockung der Filtermembran effektiv zu verhindern, die Strömung am schwächsten ist. Es muß daher eine sehr starke Crossflow-Strömung angelegt werden, um auch im Randbereich ausreichende Strömungsstärken zu erzielen, was das Verfahren wiederum energieaufwändig und daher kostenintensiv macht.

Fig. 3b zeigt das Funktionsprinzip eines Vibrationsscherkraftfiltersystems, bei dem die Verblockung der Filtermembran mit Hilfe tangentialer Vibrationen verhindert werden soll. Durch die dem System aufgezwungenen Vibrationen stellt sich ein umgekehrt parabolisches Strömungsprofil ein, mit der Folge, dass im Randbereich die höchsten Strömungsstärken erzielt werden. Auf diese Weise wird bei geringem Energieaufwand eine Verblockung der Filtermembran effektiv unterbunden.

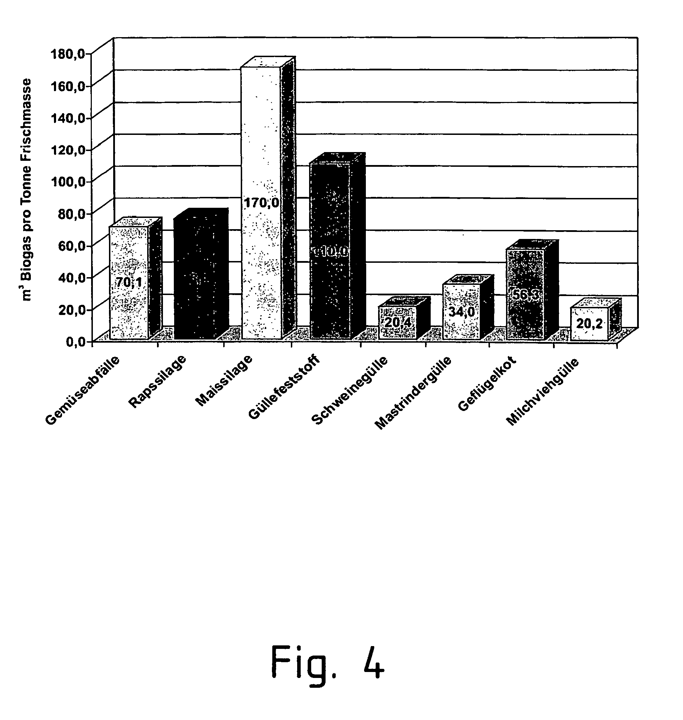

Fig. 4 zeigt die Biogasausbeuten verschiedener häufig verwendeter Gärsubstrate in einem Biogasfermenter im Vergleich zur Gasausbeute des mit dem erfindungsgemäßen Verfahren abgetrennten Güllefeststoffs aus gemischter Rinder- und Schweinegülle. Es zeigt sich, dass der Güllefeststoff eine außerordentlich hohe Biogasausbeute bringt und sich daher als kostengünstiger Ersatz für Maissilage eignet.

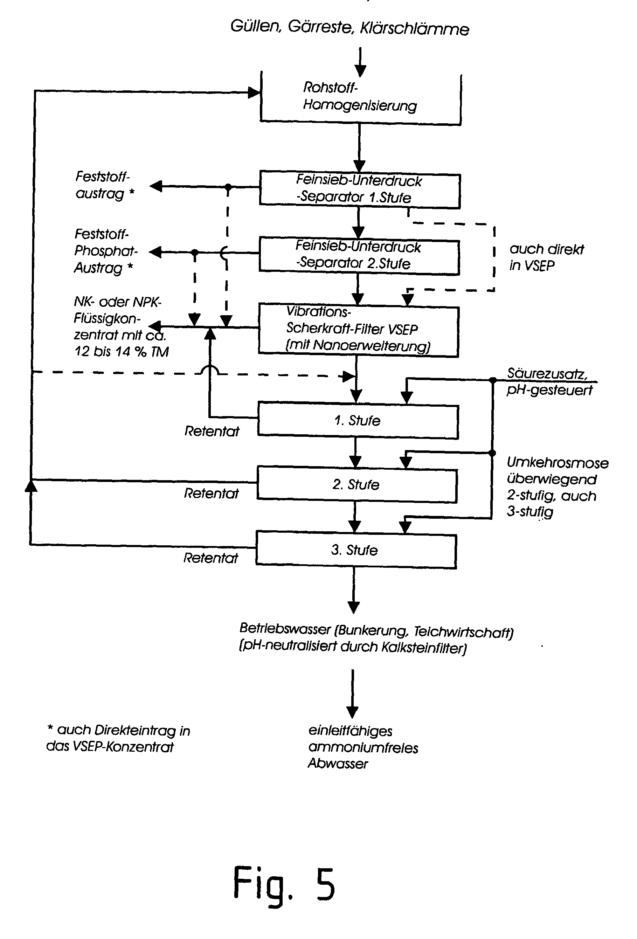

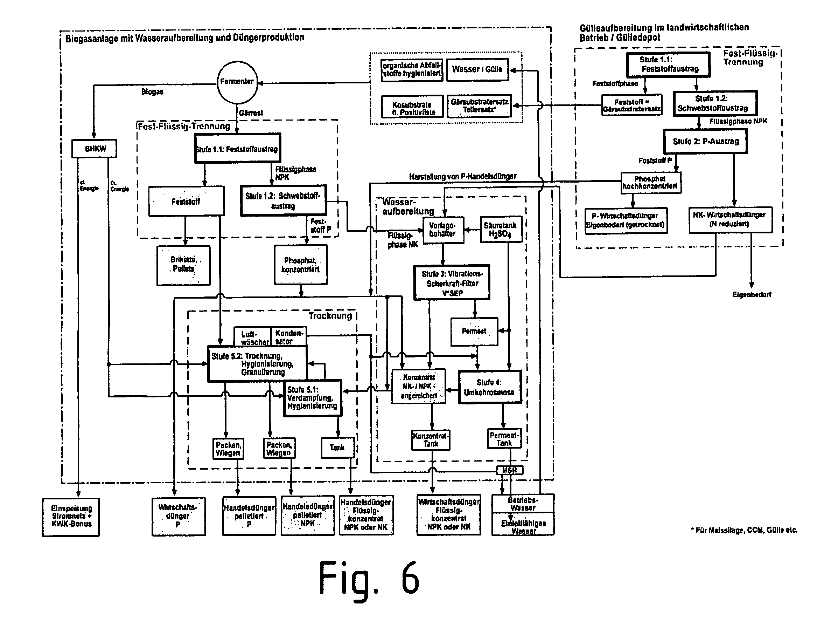

Fig. 5 gibt einen Überblick über das erfindungsgemäße Verfahren, während Fig. 6 im Überblick die Einbringung des erfindungsgemäßen Verfahrens in den Kontext einer landwirtschaftlichen Biogasanlage mit Düngeraufbereitung darstellt.

## Patentansprüche

1. Verfahren zur Aufbereitung von Klärschlämmen, Güllen und Gärresten aus der Biogasproduktion, aufweisend die folgenden Schritte:
a. Einbringen des Klärschlamms, der Gülle oder des Gärrests in einen Feinsiebunterdruckseparator (FUS), aufweisend
- eine Senkrecht-Hebewendel,
- einen die Wendel umschließenden Zylinder, der zumindest abschnittsweise ein Sieb mit einer Maschengröße zwischen 30 und 300 µm aufweist,
- sowie ein den Zylinder umgebendes Unterdruckgehäuse,
b. Herstellen und Auffangen eines Filtrats sowie eines separierten Feststoffs,
c. Einbringen des Filtrats in einen Vibrationsscherkraftfilter und
d. Herstellen und Auffangen eines primären Permeats sowie eines primären Retentats.

2. Verfahren gemäß Anspruch 1, **dadurch gekennzeichnet, dass** das Filtrat nach Passieren des Feinsiebunterdruckseparators und vor Einbringen in den Vibrationsscherkraftfilter in einen weiteren Feinsiebunterdruckseparator eingebracht wird, wobei letzterer zumindest abschnittsweise ein Sieb mit einer geringeren Maschengröße aufweist als der erstgenannte Feinsiebunterdruckseparator.

3. Verfahren gemäß Anspruch 1 oder 2, **dadurch gekennzeichnet, dass** das primäre Permeat aus dem Vibrationsscherkraftfilter einer Umkehrosmose unterzogen wird, und dass das auf diese Weise hergestellte sekundäre Permeat sowie des sekundäre Retentat aufgefangen werden.

4. Verfahren gemäß einem der vorherigen Ansprüche, **dadurch gekennzeichnet, dass** das Filtrat und/oder das bzw. die Permeate angesäuert werden.

5. Verfahren gemäß einem der vorherigen Ansprüche, **dadurch gekennzeichnet, dass** der separierte Feststoff als Bodenverbesserer, als Düngemittel, als Brennstoff, als Gärsubstrat, als Zellulose-Füllstoff, als ein Bestandteil derselben oder zur Herstellung derselben verwendet wird.

6. Verfahren gemäß einem der vorherigen Ansprüche, **dadurch gekennzeichnet, dass** das gemäß Anspruch 1 und/oder gemäß Anspruch 3 gewonnene Retentat als Düngemittel weiterverwendet bzw. zu einem Handels-Düngemittel weiterverarbeitet wird.

7. Verfahren gemäß einem der vorherigen Ansprüche, **dadurch gekennzeichnet, dass** der separierte Feststoff und das gemäß Anspruch 1 und/oder gemäß Anspruch 3 gewonnene Retentat vereinigt und als Bodenverbesserer, als Düngemittel, als Brennstoff, als Gärsubstrat, als mineralischer Dünger, als ein Bestandteil derselben oder zur Herstellung derselben verwendet wird.

8. Verfahren gemäß einem der vorherigen Ansprüche, **dadurch gekennzeichnet, dass** der separierte Feststoff und das gemäß Anspruch 1 und/oder gemäß Anspruch 3 gewonnene Permeat als Betriebswasser in einer Biogasanlage, als Reinigungs- oder Kühlwasser oder als landwirtschaftliches Produktionswasser verwendet und/oder in einen Vorfluter, einen Bach, einen Wasserlauf oder eine Kanalisation eingeleitet wird.

9. Vorrichtung zur Durchführung eines Verfahrens gemäß einem der Ansprüche 1 - 8, aufweisend:
a. mindestens einen Feinsiebunterdruckseparator (FUS), aufweisend
- eine Senkrecht-Hebewendel,
- einen die Wendel umschließenden Zylinder, der zumindest abschnittsweise ein Sieb mit einer Maschengröße zwischen 30 und 300 µm aufweist,
- sowie ein den Zylinder umgebendes Unterdruckgehäuse, zur Auftrennung von Klärschlamm, Gülle oder eines Gärrests in einen Feststoff und ein Filtrat, sowie
b. einen Vibrationsscherkraftfilter zur Auftrennung des Filtrats in ein erstes Permeat und ein erstes Retentat.

10. Vorrichtung gemäß Anspruch 9, **dadurch gekennzeichnet, dass** mindestens ein weiterer Feinsiebunterdruckseparator vorgesehen ist, der zumindest abschnittsweise ein Sieb mit einer geringeren Maschengröße aufweist als der erstgenannte Feinsiebunterdruckseparator.

11. Vorrichtung gemäß Anspruch 9 oder 10, **dadurch gekennzeichnet, dass** eine Umkehrosmoseeinrichtung zur Auftrennung des ersten Permeats in ein zweites Permeat und ein zweites Retentat vorgesehen ist.

## Claims

1. A method for the treatment of waste water sludges, slurries and fermentation residues from biogas production, comprising the following steps:
a. Introducing the sewage sludge, the slurry or the fermentation residue in a fine screen vacuum separator (FUS) having
- a vertical lifting coil,
- a cylinder enclosing the coil, which at least by sections comprises a screen with a mesh size between 30 and 300 µm
- as well as a vacuum housing surrounding the cylinder,
b. producing and collecting a filtrate as well as a separated solid,
c. introducing the filtrate into a vibrating shear force filter and
d. producing and collecting a primary permeate as well as a primary retentate.

2. The method according to Claim 1, **characterized in that** the filtrate having passed the fine screen vacuum separator and before introduction into the vibrating shear force filter is introduced into a further fine screen vacuum separator, wherein the latter at least by sections comprises a screen with a lesser mesh size than the fine screen vacuum separator mentioned first.

3. The method according to Claim 1 or 2, **characterized in that** the primary permeate from the vibrating shear force filter is subjected to a reverse osmosis and that the secondary permeate as well as the secondary retentate produced in this manner are collected.

4. The method according to any one of the preceding claims, **characterized in that** the filtrate and/or the permeate or permeates are acidified.

5. The method according to any one of the preceding claims, **characterized in that** the separated solid is used as soil improver, as fertilizer, as fuel, as fermentation substrate, as cellulose filler, as a part of the same or for the production of the same.

6. The method according to any one of the preceding claims, **characterized in that** the retentate derived according to Claim 1 and/or according to Claim 3 is further used as fertilizer or further processed into a commercial fertilizer.

7. The method according to any one of the preceding claims, **characterized in that** the separated solid and the retentate derived according to Claim 1 and/or according to Claim 3 are combined and used as soil improver, as fertilizer, as fuel, as fermentation substrate, as mineral fertilizer, as a part of the same or for the production of the same.

8. The method according to any one of the preceding claims, **characterized in that** the separated solid and the permeate derived according to Claim 1 and/or according to Claim 3 are used as process water in a biogas plant, as cleaning or cooling water or as agricultural production water and/or discharged into a receiving water, a brook, a water course or a drain.

9. A device to carry out a method according to any one of the Claims 1 - 8, comprising
a. at least one fine screen vacuum separator (FUS), comprising
- a vertical lift coil
- a cylinder enclosing the coil which at least by sections comprises a screen with a mesh size between 30 and 300 µm,
- as well as a vacuum housing surrounding the cylinder for the separation of sewage sludge, slurry or fermentation residue into a solid and a filtrate, as well as
b. a vibrating shear force filter for separating the filtrate into a first permeate and a first retentate.

10. The device according to Claim 9, **characterized in that** at least one further fine screen vacuum separator is provided, which at least by sections comprises a screen with a lesser mesh size than the fine screen vacuum separator mentioned first.

11. The device according to Claim 9 or 10, **characterized in that** a reverse osmosis device for separating the first permeate into a second permeate and a second retentate is provided.

## Revendications

1. Procédé pour la préparation de boues d'épuration, de lisiers et de résidus de fermentation à partir de la production de biogaz, comportant les étapes suivantes :
a. Introduction de la boue d'épuration, du lisier ou des résidus de fermentation dans un séparateur sous pression à micro-tamis (FUS), comprenant :
- une spirale de levage vertical,
- un cylindre entourant la spirale, comportant au moins par tronçons un tamis avec un maillage entre 30 et 300 µm,
- ainsi qu'un carter sous pression entourant le cylindre.
b. Production et captage d'un filtrat ainsi que d'un corps solide séparé,
c. Introduction du filtrat dans un filtre à force de cisaillement par vibrations, et
d. Production et captage d'un perméat primaire ainsi que d'un rétentat primaire.

2. Procédé selon la revendication 1, **caractérisé en ce que** le filtrat, après avoir franchi le séparateur sous pression à micro-tamis et avant l'insertion dans le filtre à force de cisaillement par vibrations, est introduit dans un autre séparateur sous pression à micro-tamis, ce dernier comportant au moins par tronçons un tamis avec un maillage inférieur à celui du séparateur sous pression à micro-tamis cité en premier.

3. Procédé selon la revendication 1 ou 2, **caractérisé en ce que** le perméat primaire venant du filtre à force de cisaillement par vibrations est soumis à une osmose inverse, et **en ce que** le perméat secondaire produit de cette manière ainsi que le rétentat secondaire sont captés.

4. Procédé selon l'une des revendications précédentes, **caractérisé en ce que** le filtrat et/ou le ou les perméats sont acidifiés.

5. Procédé selon l'une des revendications précédentes, **caractérisé en ce que** le corps solide séparé est utilisé comme engrais, comme combustible, comme substrat de fermentation, comme agent de charge de cellulose, comme composant de ceux-ci ou pour la fabrication de ceux-ci.

6. Procédé selon l'une des revendications précédentes, **caractérisé en ce que** le rétentat produit selon la revendication 1 et/ou selon la revendication 3 est réutilisé comme engrais ou traité pour en faire un engrais commercialisé.

7. Procédé selon l'une des revendications précédentes, **caractérisé en ce que** le corps solide séparé et le rétentat produit selon la revendication 1 et/ou selon la revendication 3 sont combinés et utilisés comme amendement, comme engrais, comme combustible, comme substrat de fermentation, comme engrais minéral, comme composant de ceux-ci ou pour la fabrication de ceux-ci.

8. Procédé selon l'une des revendications précédentes, **caractérisé en ce que** le corps solide séparé et le perméat produit selon la revendication 1 et/ou selon la revendication 3 sont utilisés comme eau industrielle dans une usine de production de biogaz, comme eau de nettoyage ou de refroidissement ou comme eau de production agricole et/ou déversés dans un cours d'eau récepteur, une rivière, un cours d'eau ou une canalisation.

9. Dispositif pour la mise en oeuvre d'un procédé selon l'une des revendications 1 à 8, comprenant :
a. au moins un séparateur sous pression à micro-filtre (FUS), comportant
- une spirale de levage vertical,
- un cylindre entourant la spirale, comportant au moins par tronçons un tamis avec un maillage entre 30 et 300 µm,
- ainsi qu'un carter sous pression entourant le cylindre, pour la séparation de boues d'épuration, de lisiers ou de résidus de fermentation dans un corps solide ou un filtrat, et
b. un filtre à force de cisaillement par vibrations pour la séparation du filtrat en un premier perméat et un premier rétentat.

10. Dispositif selon la revendication 9, **caractérisé en ce qu'**il est prévu au moins un autre séparateur sous pression à micro-filtre, comportant au moins par tronçons un tamis avec un maillage inférieur à celui du séparateur sous pression à micro-filtre cité en premier.

11. Dispositif selon la revendication 9 ou 10, **caractérisé en ce qu'**il est prévu une installation à osmose inverse, pour la séparation du premier perméat en un deuxième perméat et un deuxième rétentat.
